# DEMANDE DE BREVET EUROPEEN

(11) **EP 0 745 691 A2**
(43) Date de publication de la demande: **04.12.1996**
(21) Numéro de dépôt: 96420152.9
(22) Date de dépôt: 02.05.1996
(51) Int. Cl.: C12Q 1/68, C12P 19/34, C07H 21/04

(54) **Fragment nucléotidique de l'ARN ribosomique 16s de corynébactéries, sondes et amorces dérivées, réactif et procédé de détection**

(30) Priorité: 03.05.1995 FR 9505494
(71) Demandeur: BIO MERIEUX, F-69280 Marcy l'Etoile (FR)
(72) Inventeur: Mabilat, Claude, 69140 Rillieux la Pape (FR); Ruimy, Raymond, 06200 Nice (FR)
(74) Mandataire: Guerre, Dominique

(57) **Abrégé**

L'invention concerne des fragments nucléotidiques monocaténaires de l'ARN ribosomique 16S des espèces pathogènes du genre *Corynebacterium*, choisis parmi les fragments présentant les séquences nucléotidiques de l'ARN 16S constitué par les nucléotides respectivement :
commençant au nucléotide N°39 et finissant au nucléotide N°67
commençant au nucléotide N°162 et finissant au nucléotide N°182
commençant au nucléotide N°433 et finissant au nucléotide N°461
commençant au nucléotide N°575 et finissant au nucléotide N°598
commençant au nucléotide N°805 et finissant au nucléotide N°820
commençant au nucléotide N°826 et finissant au nucléotide N°842
commençant au nucléotide N°980 et finissant au nucléotide N°1000
et leurs séquences complémentaires.

L'invention concerne en outre les sondes, les amorces dont les séquences appartiennent à celles du fragment de l'invention, un réactif et un procédé pour identifier les espèces pathogènes de corynébactéries.

## Description

La présente invention relève du domaine des techniques de détection et/ou d'amplification de matériel nucléique, à l'aide de sondes ou d'amorces oligonucléotidiques, et leur application notamment à la recherche de la présence ou à l'identification de bactéries du genre *Corynebacterium*.

Les corynébactéries sont des bactéries commensales constituant un groupe hétérogène sur les plans morphologique, taxonomique, pathogénique et thérapeutique. Cette hétérogénéité a longtemps conduit à une méconnaissance de ce groupe et limité l'identification des différentes souches. Chez l'homme, la pathogénicité des espèces appartenant au genre *Corynebacterium* est très variable, et de nombreuses corynébactéries font partie de la flore normale de la peau et des muqueuses. Ainsi, sur le plan pathogénique, certaines souches sont hautement pathogènes pour l'homme (*Corynebacterium diphtheriae)*, mais la plupart sont des bactéries opportunistes, responsables d'infections chez des patients immunodéprimés ou affaiblis. En effet, d'un point de vue épidémiologique, le pouvoir pathogène des corynébactéries a pendant longtemps été limité à la diphtérie (*Corynebacterium diphtheriae)* mais cette maladie est actuellement en régression, en raison notamment des mesures de vaccination obligatoire, et a laissé la place à de nouvelles pathologies liées à l'évolution de la médecine hospitalière. Ainsi, les corynébactéries sont de plus en plus fréquemment isolées dans le cadre d'infections nosocomiales.

Ces infections à corynébactéries (voir tableau ci-après) survenant essentiellement chez des patients très affaiblis, il est important de disposer d'un test de diagnostic bactériologique suffisamment spécifique et sensible pour permettre une détection rapide et sélective de l'espèce responsable, afin de mettre en oeuvre le plus rapidement possible une thérapie appropriée. Or, actuellement, les laboratoires d'analyses ne disposent que de tests biochimiques et bactériologiques, limités par des étapes d'isolement et de culture bactérienne indispensables aux tests, et qui souvent ne sont pas suffisamment spécifiques et sensibles pour diagnostiquer la présence d'une espèce déterminée de corynébactéries, et plus particulièrement d'une espèce pathogène, dans un échantillon biologique.

La présente invention pallie les inconvénients précédemment cités pour la mise en évidence de la présence de bactéries du genre *Corynebacterium*, et plus particulièrement des espèces pathogènes, par utilisation d'un marqueur génétique dans un procédé de détection par hybridation d'acides nucléiques, alliant spécificité, sensibilité et rapidité.

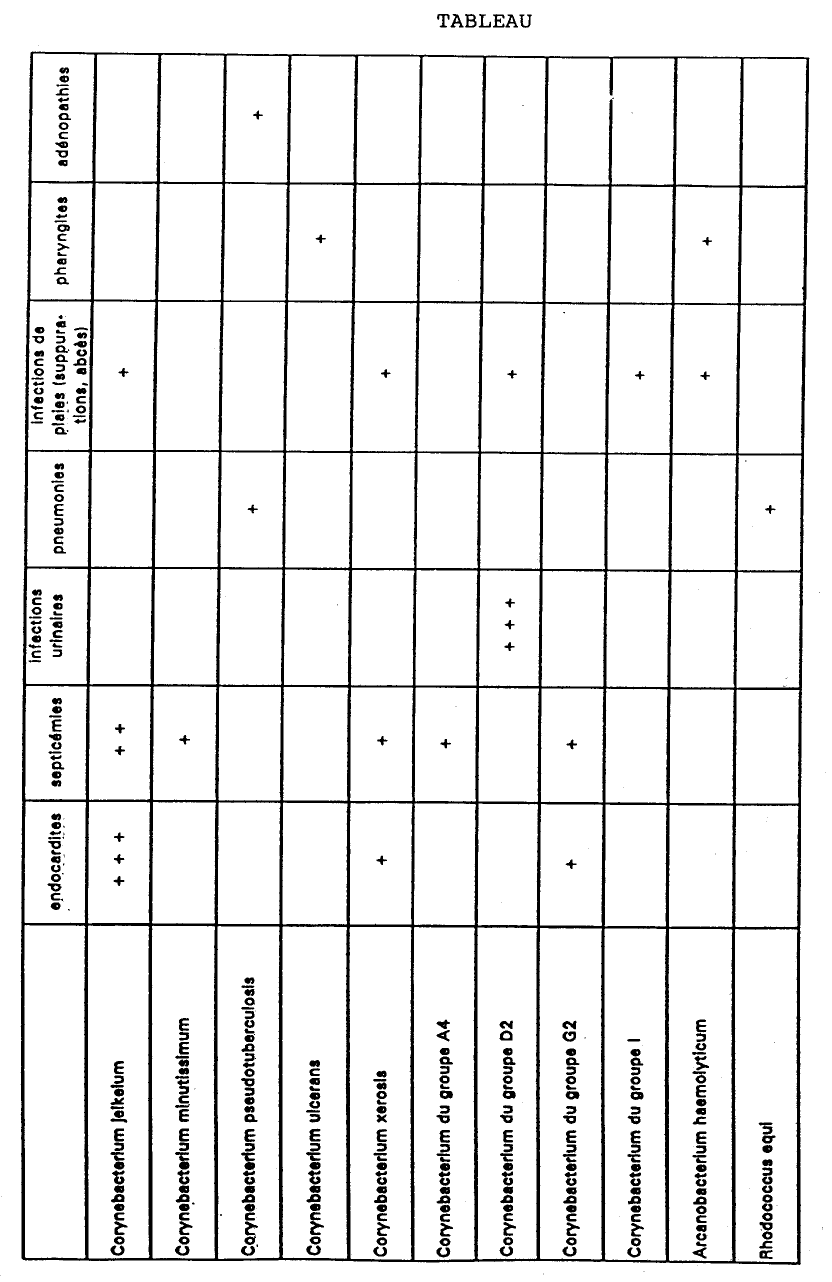

Avant d'exposer l'invention, différents termes utilisés dans la description et les revendications sont à présent définis :
- par espèce pathogène appartenant au genre *Corynebacterium*, on comprend les espèces susceptibles de provoquer, chez l'homme ou chez l'animal, une infection ou une surinfection, par opposition aux espèces du même genre intégrant la flore normale de la peau et des muqueuses ; est également considérée comme une espèce pathogène toute espèce qui n'induit pas un état pathologique chez un patient tel qu'un patient sain, mais provoque des lésions chez un autre patient, tel qu'un patient immuno-déprimé ; en particulier certaines espèces non pathogènes chez le sujet sain, sont rencontrées dans le milieu hospitalier où séjournent des patients immuno-déprimés, chez qui ces mêmes espèces sont pathogènes ;
- par "acide nucléique extrait de bactéries" on entend soit l'acide nucléique total, soit l'ARN ribosomique, en particulier l'ARNr 16S, soit l'ADN génomique, soit encore l'ADN obtenu à partir de la transcription inverse de l'ARN ribosomique, en particulier l'ARN 16S ;
- selon l'invention, un fragment nucléotidique ou un oligonucléotide est un enchaînement de monomères, caractérisé par la séquence informationnelle des acides nucléiques naturels, susceptibles de s'hybrider à un fragment nucléotidique dans des conditions prédéterminées, l'enchaînement pouvant contenir des monomères de structures différentes et être obtenu à partir d'une molécule d'acide nucléique naturelle et/ou par recombinaison génétique et/ou par synthèse chimique ;
- ainsi un monomère peut être un nucléotide naturel d'acide nucléique dont les éléments constitutifs sont un sucre, un groupement phosphate et une base azotée ; dans l'ARN le sucre est le ribose, dans l'ADN le sucre est le désoxy-2-ribose ; selon qu'il s'agisse de l'ADN ou l'ARN, la base azotée est choisie parmi l'adénine, la guanine, l'uracile, la cytosine, la thymine; ou un nucléotide modifié dans l'un au moins des trois éléments constitutifs ; à titre d'exemple, la modification peut intervenir au niveau des bases, générant des bases modifiées telles que l'inosine, la méthyl-5-désoxycytidine, la désoxyuridine, la diméthylamino-5-désoxyuridine, la diamino-2,6-purine, la bromo-5-désoxyuridine et toute autre base modifiée favorisant l'hybridation, au niveau du sucre à savoir le remplacement d'au moins un désoxyribose par un polyamide (P.E. Nielsen et al, Science, 254, 1497-1500 (1991)), au niveau du groupement phosphate par exemple son remplacement par des esters notamment choisis parmi les esters de diphosphate, d'alkyl et arylphosphonate et de phosphorothioate ;
- par "séquence informationnelle", on entend toute suite ordonnée de monomères, dont la nature chimique et l'ordre dans un sens de référence constituent une information de même qualité que celle des acides nucléiques naturels ;
- par "hybridation", on entend le processus au cours duquel, dans des conditions appropriées, deux fragments nucléotidiques ayant des séquences suffisamment complémentaires sont susceptibles de s'associer par des liaisons hydrogène stables et spécifiques, pour former un double brin. Les conditions d'hybridation sont déterminées par la "stringence", c'est-à-dire la rigueur des conditions opératoires ; l'hybridation est d'autant plus spécifique qu'elle est effectuée à plus forte stringence ; la stringence est fonction notamment de la composition en bases du duplex sonde/cible, ainsi que du degré de mésappariement entre deux acides nucléiques ; la stringence peut également être fonction des paramètres de la réaction d'hybridation, tels que la concentration et le type d'espèces ioniques présentes dans la solution d'hybridation, la nature et la concentration d'agents dénaturants et/ou la température d'hybridation ; la stringence des conditions dans lesquelles une réaction d'hybridation doit être réalisée dépend notamment des sondes utilisées ; toutes ces données sont bien connues de l'homme du métier, et les conditions appropriées peuvent éventuellement être déterminées dans chaque cas par des expériences de routine ; en général, selon la longueur des sondes utilisées, la température pour la réaction d'hybridation est comprise entre environ 20 et 65°C, en particulier entre 35 et 65°C, dans une solution saline à une concentration d'environ 0,8 à 1M ;
- une sonde est un fragment nucléotidique comprenant au moins 5 monomères, avantageusement au moins 8 monomères, possédant une spécificité d'hybridation dans des conditions déterminées pour former un complexe d'hybridation avec un fragment nucléotidique ayant une séquence nucléotidique déterminée comprise dans l'ARN ribosomique, ou l'ADN obtenu par transcription inverse dudit ARN ribosomique, ou l'ADN dont ledit ARN ribosomique est le produit de transcription ; une sonde peut être utilisée à des fins de diagnostic, et être dans ce cas une sonde de capture et/ou de détection, ou à des fins de thérapie ;
- la sonde de capture peut être immobilisée sur un support solide par tout moyen approprié, c'est-à-dire directement ou indirectement, par exemple par covalence ou adsorption passive ;
- la sonde de détection est marquée au moyen d'un marqueur choisi parmi les isotopes radioactifs, des enzymes notamment choisis parmi la peroxydase et la phosphatase alcaline et ceux susceptibles d'hydrolyser un substrat chromogène, fluorigène ou luminescent, des composés chimiques chromophores, des composés chromogènes, fluorigènes ou luminescents, des analogues de bases nucléotidiques, et la biotine ;
- les sondes utilisées à des fins de diagnostic de l'invention peuvent être mises en oeuvre dans toutes les techniques d'hybridation connues et notamment les techniques dites "DOT-BLOT" (MANIATIS et al, Molecular Cloning, Cold Spring Harbor, 1982), "SOUTHERN BLOT" (SOUTHERN. E.M., J. Mol. Biol., 98, 503 (1975)), "NORTHERN BLOT" qui est une technique identique à la technique "SOUTHERN BLOT" mais qui utilise de l'ARN comme cible, la technique SANDWICH (DUNN A.R., HASSEL J.A., Cell, 12, 23 (1977)) ; avantageusement, on utilise la technique SANDWICH dans la présente invention comprenant une sonde de capture spécifique et/ou une sonde de détection spécifique, étant entendu que la sonde de capture et la sonde de détection doivent présenter une séquence nucléotidique au moins partiellement différente ;
- une autre application de l'invention est une sonde de thérapie pour traiter les infections dues aux corynébactéries, ladite sonde étant susceptible de s'hybrider in vivo sur l'ARN ribosomique 16S desdites bactéries et/ou sur l'ADN génomique, pour bloquer les phénomènes de traduction et/ou de transcription ;
- une amorce est une sonde comprenant de 5 à 30 monomères, possédant une spécificité d'hybridation dans des conditions déterminées pour l'initiation d'une polymérisation enzymatique, par exemple dans une technique d'amplification telle que la PCR (Polymerase Chain Reaction), dans un procédé d'élongation, tel que le séquençage, dans une méthode de transcription inverse ou analogue ;
- l'homologie caractérise le degré d'identité de deux fragments nucléotidiques comparés ; elle se mesure par le pourcentage d'identité qui est notamment déterminé par comparaison directe de séquences nucléotidiques par rapport à des séquences nucléotidiques de référence ;
- tout fragment nucléotidique est dit équivalent ou dérivé d'un fragment de référence, s'il présente une séquence nucléotidique équivalente à la séquence de référence, tel que :
   a) tout fragment susceptible de s'hybrider au moins partiellement avec le complément du fragment de référence,
   b) tout fragment dont l'alignement avec le fragment de référence conduit à mettre en évidence des bases contigües identiques en nombre plus important qu'avec toute autre fragment provenant d'un autre groupe taxonomique,
   c) tout fragment résultant ou pouvant résulter de la variabilité naturelle de l'espèce à partir de laquelle il est obtenu,
   d) tout fragment pouvant résulter des techniques de génie génétique appliquées au fragment de référence,
   e) tout fragment différent du fragment de référence, par insertion, délétion, substitution d'au moins un monomère, extension, ou raccourcissement à l'une au moins de ses extrémités.

En particulier, les sondes et amorces selon la présente invention, sont celles dont les séquences sont identiques ou équivalentes à l'une des séquences identifiées ci-après, notamment les séquences présentant au moins 70 % d'homologie avec l'une desdites séquences et une suite d'au moins 5 monomères contigus appartenant à cette séquence.

Selon l'invention on a déterminé des fragments nucléotidiques monocaténaires de l'ARN ribosomique 16S appartenant à des régions spécifiques d'au moins une espèce de corynébactéries. Pour 21 espèces pathogènes de corynébactéries, ces fragments sont représentés dans la liste des séquences donnée en fin de description, et identifiés par leur numéro SEQ ID NO 1 à 91. Ces 21 espèces pathogènes sont les suivantes :
*Corynebacterium afermentans afermantans*
*Corynebacterium afermantans lipophilum*
*Corynebacterium amycolatum*
*Corynebacterium argentoratense*
*Corynebacterium asperum*
*Corynebacterium bovis*
*Corynebacterium cystitidis*
*Corynebacterium diphtheriae*
*Corynebacterium flavescens*
*Corynebacterium jeikeium*
*Corynebacterium matruchotii*
*Corynebacterium minutissimum*
*Corynebacterium mycetoides*
*Corynebacterium pilosum*
*Corynebacterium pseudodiphteriticum*
*Corynebacterium pseudotuberculosis*
*Corynebacterium seminale*
*Corynebacterium sp group F-1*
*Corynebacterium striatum*
*Corynebacterium ulcerans*
*Corynebacterium urealyticum*

Les fragments de l'invention sont définis par une numérotation des nucléotides en utilisant comme référence la numérotation de la séquence nucléotidique de l'ARN ribosomique 16S de *Corynebacterium diphtheriae*, complètement identifiée par le déposant, et décrite en fin de description sous la référence SEQ ID NO 92.

Les fragments nucléotidiques monocaténaires de l'invention présentent une séquence nucléotidique choisie parmi les séquences suivantes (numérotation en référence à la SEQ ID NO 92) :
commençant au nucléotide N°39 et finissant au nucléotide N°67
commençant au nucléotide N°162 et finissant au nucléotide N°182
commençant au nucléotide N°433 et finissant au nucléotide N°461
commençant au nucléotide N°575 et finissant au nucléotide N°598
commençant au nucléotide N°805 et finissant au nucléotide N°820
commençant au nucléotide N°826 et finissant au nucléotide N°842
commençant au nucléotide N°980 et finissant au nucléotide N°1000 de la sous-unité 16S de l'ARN ribosomique de toute espèce pathogène appartenant au genre *Corynebacterium*, selon le séquençage de ladite sous-unité de *Corynebacterium diphtheriae*, et leurs séquences complémentaires.

Plus particulièrement, les séquences nucléotidiques des fragments de l'invention sont choisies parmi les séquences SEQ ID NO 1 à SEQ ID NO 91 décrites en fin de description et leurs séquences complémentaires.

Conformément à la définition ci-dessus les fragments de l'invention sont des fragments d'ARN, mais ils peuvent aussi être des fragments monocaténaires d'ADN résultant de la transcription inverse des fragments d'ARN précités, ou leurs fragments complémentaires, ainsi que des fragments monocaténaires d'ADN génomique dont les produits de transcription sont les fragments d'ARN précités, ou leurs fragments complémentaires.

Un autre objet selon la présente invention sont des sondes ou des amorces nucléiques spécifiques d'espèces pathogènes de corynebactéries.

Les sondes préférentielles sont celles présentant une séquence comprise dans les séquences (numérotation en référence à SEQ ID NO 92) :
commençant au nucléotide N°39 et finissant au nucléotide N°67
commençant au nucléotide N°162 et finissant au nucléotide N°182
commençant au nucléotide N°433 et finissant au nucléotide N°461
commençant au nucléotide N°575 et finissant au nucléotide N°598
commençant au nucléotide N°805 et finissant au nucléotide N°820
commençant au nucléotide N°826 et finissant au nucléotide N°842
commençant au nucléotide N°980 et finissant au nucléotide N°1000
et leurs séquences complémentaires,
et plus particulièrement, une séquence comprise dans les séquences identifiées SEQ ID NO 1 à SEQ ID NO 91 et leurs séquences complémentaires.

Un autre objet de l'invention est une amorce pour la transcription inverse spécifique d'une séquence d'ARN ribosomique 16S des corynébactéries, en une séquence d'ADN complémentaire, ou une amorce notamment pour l'amplification spécifique, par réaction de polymérisation en chaîne, de la séquence d'ADN complémentaire à une séquence d'ARN ribosomique 16S de corynébactéries pathogènes.

L'intérêt des sondes et amorces de l'invention s'est en outre avéré quand on a observé qu'elles sont spécifiques des espèces pathogènes de corynébactéries par rapport à des espèces appartenant à un genre différent de *Corynebacterium* mais phylogéniquement proches des corynébactéries.

L'invention concerne aussi un réactif pour détecter et/ou identifier au moins une espèce pathogène de corynébactéries dans un échantillon biologique comprenant au moins une sonde de l'invention, et en particulier une sonde de capture et une sonde de détection, l'une et/ou l'autre répondant à la définition d'une sonde selon l'invention. Le réactif peut comprendre un mélange de sondes de l'invention dans le but de détecter au moins deux espèces pathogènes de corynébactéries.

Enfin, l'invention apporte un procédé pour détecter sélectivement et/ou identifier une espèce pathogène de corynébactéries dans un échantillon biologique selon lequel on expose l'ARN ribosomique 16S extrait des bactéries présentes dans l'échantillon, et éventuellement dénaturé, ou l'ADN génomique extrait et dénaturé des bactéries, ou l'ADN obtenu à partir de la transcription inverse de l'ARN ribosomique 16S, à au moins une sonde de l'invention et on détecte l'hybridation de ladite sonde.

De manière préférentielle, avant d'exposer l'ADN à la sonde de l'invention, on réalise une amplification de cet ADN en présence d'un système enzymatique adapté et au moins une amorce d'amplification de l'invention et éventuellement une amorce eubactérienne.

Les objets de l'invention et certaines de leurs applications sont exposés ci-après avec les Exemples 1 et 2.

### EXEMPLE 1

Mise en évidence de la spécificité des sondes et amorces de l'invention par rapport à 22 espèces phylogéniquement proches des corynébactéries.

Pour 22 espèces n'appartenant pas au genre *Corynebacterium*, on a déterminé des fragments de l'ARN ribosomique 16S, correspondant aux fragments de l'invention.

Les espèces sont les suivantes : *D. maris, D. sp.* @*1, D. sp.* @*2, D. sp.* @*3*, *G*. *aichiensis, G. bronchialis* @*1, G. sputi* @*1, G. terrae* @*1, M. tuberculosis* @*2, N. asteroides* @*1, N. brasiliensis, N. carnea, N. nova, N. seriolae, N. sp.* @*1 , R. equi* @*1, R. erythropolis* @*1, R. fascians* @*1, R. globerulus* @*1, R. luteus, R. rhodochrous* @*1, T. paurometabolum* @*1*.

Ces fragments ont été déterminés à partir du séquençage complet de l'ARN ribosomique 16S de ces espèces.

L'acide nucléique total des souches utilisées a été isolé par la méthode de Sjöbring et al. (1990. Polymerase chain reaction for detection of *Mycobacterium tuberculosis*. J. Clin. Microbiol. 28(10):2200-2204). Des produits d'amplification PCR qui couvrent 90% de la séquence ont été générés à partir de l'ARN ribosomique à l'aide d'amorces d'amplification de spécificité eubactérienne.

Les produits d'amplification obtenus ont été directement séquençés par la méthode de terminaison de chaîne (Sanger et al., Proc. Natl. Acad. Sci. USA, 1977, 74: 5463-5467), par cyclisation thermique utilisant une Taq ADN polymérase thermostable (Perkin).

Un alignement des fragments obtenus avec les fragments ainsi déterminés met en évidence le caractère spécifique des fragments de l'invention.

### EXEMPLE 2

### Utilisation d'une sonde spécifique dirigée contre l'ARN ribosomique 16S pour l'identification de Corynebacterium striatum

On a testé la spécificité de la sonde correspondant au brin complémentaire du fragment commençant au nucléotide N° 6 et finissant au nucléotide N° 22 de la SEQ ID NO 18, pour les souches de *Corynebacterium striatum*. Une collection de souches de corynébactéries a été testée par hybridation sur l'ARN ribosomique 16S et a permis de constater a *posteriori* la spécificité de cette sonde.

L'hybridation des ARN ribosomiques d'une bactériecible a été conduite selon le procédé de détection non-radioactif et semi-automatisé décrit dans le brevet français n° 90 07249. Une sonde de capture S8L correspondant à une sonde de spécificité eubactérienne (décrite dans la demande de brevet FR n° 93 02127) et un conjugué spécifique de détection oligonucléotide (correspondant à la sonde définie au début de l'exemple 2) - enzyme sont utilises. La manipulation a été conduite dans une plaque de microtitration selon le protocole suivant.

L'ARN ribosomique des souches a été extrait selon le protocole de base pour l'extraction de l'ARN des bactéries à Gram positif décrit dans "Current Protocols in Molecular Biology" 1987, Ausubel FM et al., Wiley interscience, New York. Dans une plaque de microtitration (Nunc 439454) est déposée une solution de 1 ng/µl de l'oligonucléotide de capture dont l'extrémité 5' a réagi avec le réactif Aminolink 2 (Applied Biosystems, Foster city , Californie) dans du PBS 3X (0,45 M NaCl 0,15 M phosphate de sodium pH 7,0). La plaque est incubée 2 h à 37° C puis lavée 3 fois avec 300 µl de PBST (PBS 1x, Tween 20: 0,5 ‰ (Merck 822184)). Le réactif Aminolink 2 permet d'ajouter à l'extrémité 5' de la sonde un bras comportant un groupement 6-aminohexyle. La sonde ainsi couplée à un ligand possédant un groupement polaire (amine primaire), et fixée de façon passive sur le support, procure un signal amélioré; voir la demande FR 91 09057.

La cible constituée par 10 µl d'extrait d'ARN totaux est mélangée avec 40 µl de tampon PBS saumon (PBS-3X + ADN de sperme de saumon 10 µg/ml (Sigma D 9156)). L'ensemble est ajouté dans le puits dans lequel est fixée la sonde de capture, avec 50 µl d'une solution du conjugué oligonucléotide-peroxydase, constituant la sonde de détection, à la concentration de 0,1 ng/µl en oligonucléotide dans un tampon PBS-cheval (PBS 3X + 10 % sérum de cheval (BioMérieux 55842)). La plaque est incubée 1 h à 37° C puis lavée par 3 fois avec 300 µl de tampon PBST. Dans chaque puits, on rajoute 100 µl de substrat OPD (ortho-phénylènediamine Cambridge Medical Biotechnology ref/456) dans un tampon 0,055 M acide citrique, 0,1 M monohydrogénophosphate de sodium, pH 4,93, à la concentration de 4 mg/ml, auquel on ajoute extemporanément du peroxyde d'hydrogène à 30% dilué au 1/1000. Après 20 min de réaction l'activité enzymatique est bloquée par 100 µl d'acide sulfurique 1N et la lecture est effectuée sur un appareil Axia Microreader (AXIA : marque enregistrée) (bioMérieux) à 492 nm.

Les bactéries-cibles étaient notamment les suivantes :
- 8 isolats de *Corynebacterium striatum*
- 1 souche des espèces suivantes :
   *Corynebacterium afermentans afermantans*
   *Corynebacterium afermantans lipophilum*
   *Corynebacterium amoniagenes* (non pathogène)
   *Corynebacterium amycolatum*
   *Corynebacterium argentoratense*
   *Corynebacterium asperum*
   *Corynebacterium bovis*
   *Corynebacterium callunae* (non pathogène)
   *Corynebacterium cystitidis*
   *Corynebacterium diphtheriae*
   *Corynebacterium flavescens*
   *Corynebacterium glutamicum* (non pathogène)
   *Corynebacterium jeikeium*
   *Corynebacterium matruchotii*
   *Corynebacterium minutissimum*
   *Corynebacterium mycetoides*
   *Corynebacterium pilosum*
   *Corynebacterium propinquum* (non pathogène)
   *Corynebacterium pseudodiphteriticum*
   *Corynebacterium pseudotuberculosis*
   *Corynebacterium seminale*
   *Corynebacterium sp group F-1*
   *Corynebacterium ulcerans*
   *Corynebacterium urealyticum*
- autres espèces bactériennes : *M. tuberculosis, N. asteroïdes, R. equi.*

Les résultats obtenus indiquent que la combinaison de sondes utilisée est, a posteriori, spécifique de *Corynebacterium striatum*. Elle ne présente pas de réaction croisée avec les acides nucléiques, en particulier l'ARN ribosomique, des autres espèces bactériennes. On a contrôlé que les ARN ribosomiques-cibles des autres espèces sont bien libérés lors de l'étape de lyse puisqu'ils réagissent avec une combinaison de sondes de capture et de détection dirigées contre l'ARN ribosomique 16S et de spécificité eubactérienne.

L'adaptation de la combinaison spécifique sur l'automate VIDAS (marque déposée - commercialisé par la société bioMérieux-VITEK) a été effectuée. La paroi du puits de microplaque est ici remplacée par le SPR (marque de commerce) ("Solid Phase Receptacle") qui est un support conique réalisé à partir d'un matériau vendu sous la dénomination K résine (copolymère butadiène-styrène) et commercialisé par la société bioMérieux-VITEK (USA). Les divers réactifs sont disposés dans une barrette à plusieurs cuvettes et les différentes étapes se déroulent dans le SPR qui est capable d'aspirer et de refouler les réactifs et qui fait donc office de pipette. La réaction d'hybridation sandwich décrite dans le protocole ci-dessous se produit sur la paroi interne du cône.

Sur la surface interne du SPR, est fixé passivement l'oligonucléotide de capture comportant à son extrémité 5' le ligand Aminolink 2 (Applied Biosystems-réf.400808) à une concentration de 1 ng/µl dans un volume de 315 µl d'une solution de PBS 4x (phosphate de sodium 200 mM pH 7,0, NaCl 600 mM). Après une nuit à température ambiante ou deux heures à 37°C, les cônes sont lavés 2 fois par une solution PBS Tween, puis séchés sous vide. La barrette contient dans des cuvettes les réactifs nécessaires à la détection, c'est à dire: 200 µl d'une solution à 0,1 ng/µl du conjugué de détection oligonucléotide-phosphatase alcaline, 2 fois 600 µl de solution de lavage PBS Tween et une cuvette de substrat.

Dans le premier puits de la barrette, sont déposés 10 µl de l'ARN extrait dans le même tampon que pour le protocole microplaque ci-dessus.

Après incubation 30 minutes du cône par le mélange cible plus sonde de détection, le cône est lavé 2 fois par une solution de PBS Tween. 250 µl de substrat MUP (méthyl-4 umbelliféryl phosphate) en solution dans un tampon diéthanolamine sont aspirés dans le cône, puis relachés dans une cuvette de lecture. L'appareil mesure le signal fluorescent exprimé en URF (unités relatives de fluorescence) de la cuvette.

Les résultats obtenus avec ce système sont les mêmes que ceux obtenus en microplaque.

## Revendications

1. Fragment nucléotidique monocaténaire appartenant à l'ARN ribosomique 16S des espèces pathogènes du genre *Corynebacterium*, caractérisé en ce que ledit fragment est choisi parmi les fragments présentant les séquences nucléotidiques suivantes de l'ARN 16S :
commençant au nucléotide N°39 et finissant au nucléotide N°67
commençant au nucléotide N°162 et finissant au nucléotide N°182
commençant au nucléotide N°433 et finissant au nucléotide N°461
commençant au nucléotide N°575 et finissant au nucléotide N°598
commençant au nucléotide N°805 et finissant au nucléotide N°820
commençant au nucléotide N°826 et finissant au nucléotide N°842
commençant au nucléotide N°980 et finissant au nucléotide N°1000,
et leurs séquences complémentaires, les numéros des nucléotides correspondant à leur position par rapport à la séquence nucléotidique SEQ ID NO 92 de l'ARN ribosomique 16S de *Corynebacterium diphtheriae*, utilisée comme référence.

2. Fragment selon la revendication 1, caractérisé en ce qu'il présente une séquence informationnelle choisie parmi les séquences SEQ ID NO 1 à SEQ ID NO 91, et leurs séquences complémentaires.

3. Fragment nucléotidique monocaténaire d'ADN, caractérisé en ce qu'il est obtenu par transcription inverse d'un fragment nucléotidique selon la revendication 1 ou 2, ou son fragment complémentaire.

4. Fragment nucléotidique monocaténaire d'ADN génomique caractérisé en ce que son produit de transcription est un fragment nucléotidique selon la revendication 1 ou 2, ou son fragment complémentaire.

5. Sonde pour l'identification d'espèces pathogènes de bactéries du genre *Corynebacterium*, caractérisée en ce qu'elle comprend une séquence d'au moins cinq monomères, identique ou équivalente à au moins une partie de la séquence nucléotidique d'un fragment selon l'une quelconque des revendications 1 à 4, notamment une séquence présentant à la fois au moins 70 % d'homologie avec au moins une partie dudit fragment et une suite d'au moins 5 monomères contigus appartenant à cette partie.

6. Sonde selon la revendication 5, caractérisée en ce qu'elle comprend une séquence d'au moins huit monomères.

7. Sonde de thérapie pour le traitement des infections dues à une espèce déterminée de corynébactéries, caractérisée en ce qu'elle comprend une séquence nucléotidique appartenant à la séquence d'un fragment selon l'une quelconque des revendications 1 à 4.

8. Amorce pour la transcription inverse spécifique d'une séquence d'ARN ribosomique 16S des corynébactéries, en une séquence d'ADN complémentaire, caractérisée en ce qu'elle comprend une séquence nucléotidique d'au moins cinq monomères, identique ou équivalente à au moins une partie de la séquence nucléotidique d'un fragment selon l'une quelconque des revendications 1 à 4, notamment une séquence présentant à la fois au moins 70 % d'homologie avec au moins une partie dudit fragment et une suite d'au moins 5 monomères contigus appartenant à cette partie.

9. Amorce notamment pour l'amplification enzymatique spécifique d'au moins une séquence d'acide nucléique, telle que par réaction de polymérisation en chaîne, caractérisée en ce qu'elle comprend une séquence d'au moins cinq monomères identique ou équivalente à au moins une partie de la séquence nucléotidique d'un fragment selon l'une quelconque des revendications 1 à 4, notamment une séquence présentant à la fois au moins 70 % d'homologie avec au moins une partie dudit fragment et une suite d'au moins 5 monomères contigus appartenant à cette partie.

10. Réactif pour détecter et/ou identifier au moins une espèce pathogène de corynébactéries dans un échantillon biologique, caractérisé en ce qu'il comprend au moins une sonde d'identification selon l'une des revendications 5 à 6.

11. Réactif selon la revendication 10, caractérisé en ce qu'il comprend une sonde dite de capture et/ou une sonde dite de détection selon la revendication 5 ou 6, ladite sonde de capture et ladite sonde de détection présentant une séquence nucléotidique au moins partiellement différente.

12. Réactif selon la revendication 11, caractérisé en ce que la sonde de capture est fixée sur un support solide directement ou indirectement.

13. Réactif selon la revendication 11 ou 12, caractérisée en ce que la sonde de détection est marquée au moyen d'un marqueur choisi parmi les isotopes radioactifs, des enzymes notamment choisis parmi la péroxydase et la phosphatase alcaline et ceux susceptibles d'hydrolyser un substrat chromogène, fluorigène ou luminescent, des composés chimiques chromophores, des composés chromogènes, fluorigènes ou luminescents, des analogues de bases nucléotidiques, et la biotine.

14. Réactif pour détecter et/ou identifier au moins deux espèces pathogènes différentes de corynébactéries dans un échantillon biologique, caractérisé en ce qu'il comprend un mélange de sondes selon l'une quelconque des revendications 5 à 6.

15. Réactif selon la revendication 10, caractérisé en ce qu'il comprend au moins une amorce selon la revendication 8 et/ou au moins une amorce selon la revendication 9.

16. Procédé de détection sélective et/ou d'identification d'une espèce pathogène de corynébactéries dans un échantillon biologique, caractérisé en ce qu'on expose l'ARN ribosomique 16S, extrait des bactéries contenues dans ledit échantillon à au moins une sonde selon la revendication 5 ou 6, et on détecte l'hybridation de ladite sonde.

17. Procédé de détection sélective et/ou d'identification d'une espèce pathogène de corynébactéries dans un échantillon biologique, caractérisé en ce qu'on expose l'ADN génomique, extrait et dénaturé des bactéries contenues dans ledit échantillon à au moins une sonde selon la revendication 5 ou 6 , puis on détecte l'hybridation de ladite sonde.

18. Procedé de détection sélective et/ou d'identification d'une espèce pathogène de corynébactéries dans un échantillon biologique, caractérisé en ce qu'on expose l'ADN obtenu par transcription inverse de l'ARN ribosomique 16S des bactéries contenues dans ledit échantillon à au moins une sonde selon la revendication 5 ou 6, puis on détecte l'hybridation de ladite sonde.

19. Procédé selon la revendication 17 ou 18, caractérisé en ce qu'avant d'exposer l'ADN à ladite sonde, on réalise une amplification dudit ADN génomique en présence d'un système enzymatique adapté et au moins une amorce selon la revendication 9.


LISTE DE SEQUENCES

(1) INFORMATIONS GENERALES:

(i) DEPOSANT:
(A) NOM: BIOMERIEUX
(B) RUE: CHEMIN DE L'ORME
(C) VILLE: MARCY L'ETOILE
(E) PAYS: FRANCE
(F) CODE POSTAL: 69280

(ii) TITRE DE L'INVENTION: Fragment nucléotidique de l'ARN
ribosomique 16S de corynébactéries, sondes et amorces dérivées,
réactif et procédé de détection.

(iii) NOMBRE DE SEQUENCES: 92

(iv) FORME DECHIFFRABLE PAR ORDINATEUR:
(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.30 (OEB)

(2) INFORMATIONS POUR LA SEQ ID NO: 1:

(i) CARACTERISTIQUES DE LA SEQUENCE:
(A) LONGUEUR: 22 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 1:

ACGGAAAGGC CUAGCUUGCU AG 22

(2) INFORMATIONS POUR LA SEQ ID NO: 2:

(i) CARACTERISTIQUES DE LA SEQUENCE:
(A) LONGUEUR: 23 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 2:

ACGGAAAGGC AGUGCUUGCA CUG 23

(2) INFORMATIONS POUR LA SEQ ID NO: 3:

(i) CARACTERISTIQUES DE LA SEQUENCE:
(A) LONGUEUR: 22 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 3:

ACGGAAAGGC CAAGCUUGCU UG 22

(2) INFORMATIONS POUR LA SEQ ID NO: 4:

(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 4:

ACGGUAAGGC UCCAGCUUGC UGGG 24

(2) INFORMATIONS POUR LA SEQ ID NO: 5:

(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 5:

ACGGAAAGGC CUAAGCUUGC UUGG 24

(2) INFORMATIONS POUR LA SEQ ID NO: 6:

(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 6:

ACGGAAAGGC CCCAGCUUGC UGGG 24

(2) INFORMATIONS POUR LA SEQ ID NO: 7:

(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 7:

ACGGAAAGGC UCUAGCUUGC UAGG 24

(2) INFORMATIONS POUR LA SEQ ID NO: 8:

(i) CARACTERISTIQUES DE LA SEQUENCE:
(A) LONGUEUR: 26 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 8:

ACGGAAAGGC CCCUAGCUUG CUGGGG 26

(2) INFORMATIONS POUR LA SEQ ID NO: 9:

(i) CARACTERISTIQUES DE LA SEQUENCE:
(A) LONGUEUR: 26 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 9:

ACGGAAAGGC UCCUUGCUUG CAAGGG 26

(2) INFORMATIONS POUR LA SEQ ID NO: 10:

(i) CARACTERISTIQUES DE LA SEQUENCE:
(A) LONGUEUR: 29 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 10:
ACGGAAAGGC CCCCACUUUU UUGGUGGGG 29
(2) INFORMATIONS POUR LA SEQ ID NO: 11:
(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire
(ii) TYPE DE MOLECULE: ARNr
(xi) DESCRIPTION DE LA SEQUENCE: SEQ ID NO: 11:
ACGGAAAGGC CGAAGCUUGC UUUG 24
(2) INFORMATIONS POUR LA SEQ ID NO: 12:
(i) CARACTERISTIQUES DE LA SEQUENCE:
(A) LONGUEUR: 21 paires de bases
(B) TYPE: nucléotide
(C) NOMBRE DE BRINS: simple
(D) CONFIGURATION: linéaire
(ii) TYPE DE MOLECULE: ARNr
(xi) DESCRIPTION DE LA SEQUENCE: SEQ ID NO: 12:
ACGGAAAGGC CUGCUUGCAG G 21
(2) INFORMATIONS POUR LA SEQ ID NO: 13:
(i) CARACTERISTIQUES DE LA SEQUENCE:
(A) LONGUEUR: 20 paires de bases
(B) TYPE: nucléotide
(C) NOMBRE DE BRINS: simple
(D) CONFIGURATION: linéaire
(ii) TYPE DE MOLECULE: ARNr
(xi) DESCRIPTION DE LA SEQUENCE: SEQ ID NO: 13:
ACGGAAAGGC CUCUUCGGAG 20
(2) INFORMATIONS POUR LA SEQ ID NO: 14:
(i) CARACTERISTIQUES DE LA SEQUENCE:
(A) LONGUEUR: 20 paires de bases
(B) TYPE: nucléotide
(C) NOMBRE DE BRINS: simple
(D) CONFIGURATION: linéaire
(ii) TYPE DE MOLECULE: ARNr

(xi) DESCRIPTION DE LA SEQUENCE: SEQ ID NO: 14:
ACGGAAAGGC CCCUUCGGGG 20

(2) INFORMATIONS POUR LA SEQ ID NO: 15:

(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 15:
ACGGAAAGGC UCCAGCUUGC UGGG 24

(2) INFORMATIONS POUR LA SEQ ID NO: 16:

(i) CARACTERISTIQUES DE LA SEQUENCE:
(A) LONGUEUR: 25 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 16:
ACGGAAAGGC UCCUGCUUGC UAGGG 25

(2) INFORMATIONS POUR LA SEQ ID NO: 17:

(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 17:
ACGGAAAGGC AGUGCUUGCU ACUG 24

(2) INFORMATIONS POUR LA SEQ ID NO: 18:

(i) CARACTERISTIQUES DE LA SEQUENCE:
(A) LONGUEUR: 25 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 18:

ACGGAAAGGC CGCAGCUUGC UGCGG 25

(2) INFORMATIONS POUR LA SEQ ID NO: 19:

(i) CARACTERISTIQUES DE LA SEQUENCE:
(A) LONGUEUR: 19 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 19:

ACGGAAAGGC CCCUUCGGG 19

(2) INFORMATIONS POUR LA SEQ ID NO: 20:

(i) CARACTERISTIQUES DE LA SEQUENCE:
(A) LONGUEUR: 23 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 20:

ACGGAAAGGC CCCUGCUUGC AGG 23

(2) INFORMATIONS POUR LA SEQ ID NO: 21:

(i) CARACTERISTIQUES DE LA SEQUENCE:
(A) LONGUEUR: 18 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 21:

CAUGCUUUAG UGUGUGUG 18

(2) INFORMATIONS POUR LA SEQ ID NO: 22:

(i) CARACTERISTIQUES DE LA SEQUENCE:
(A) LONGUEUR: 18 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 22:

CACAUUUUGG AUGGUGUG 18

(2) INFORMATIONS POUR LA SEQ ID NO: 23:

(i) CARACTERISTIQUES DE LA SEQUENCE:
(A) LONGUEUR: 18 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 23:

CAUGGUGUGG AUGCUGUG 18

(2) INFORMATIONS POUR LA SEQ ID NO: 24:

(i) CARACTERISTIQUES DE LA SEQUENCE:
(A) LONGUEUR: 19 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 24:

CAUCCUUUAG UGUGUGAUG 19

(2) INFORMATIONS POUR LA SEQ ID NO: 25:

(i) CARACTERISTIQUES DE LA SEQUENCE:
(A) LONGUEUR: 17 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 25:

CGCACCGUGA GGGUGUG 17

(2) INFORMATIONS POUR LA SEQ ID NO: 26:

(i) CARACTERISTIQUES DE LA SEQUENCE:
(A) LONGUEUR: 18 paires de bases
(B) TYPE: nucléotide
(C) NOMBRE DE BRINS: simple

(D) CONFIGURATION: linéaire

(ii) TYPE DE MOLECULE: ARNr

(xi) DESCRIPTION DE LA SEQUENCE: SEQ ID NO: 26:

CGUGCUUUAG UGUGUGCG 18

(2) INFORMATIONS POUR LA SEQ ID NO: 27:

(i) CARACTERISTIQUES DE LA SEQUENCE:
(A) LONGUEUR: 17 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 27:

GUAUCUUUUG UGGGUGU 17

(2) INFORMATIONS POUR LA SEQ ID NO: 28:

(i) CARACTERISTIQUES DE LA SEQUENCE:
(A) LONGUEUR: 17 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 28:

GGUCUUUGGU GUGAUUG 17

(2) INFORMATIONS POUR LA SEQ ID NO: 29:

(i) CARACTERISTIQUES DE LA SEQUENCE:
(A) LONGUEUR: 19 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 29:

CAUGGUUUAG UGUCUCAUG 19

(2) INFORMATIONS POUR LA SEQ ID NO: 30:

(i) CARACTERISTIQUES DE LA SEQUENCE:
(A) LONGUEUR: 18 paires de bases
(B) TYPE: nucléotide

(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 30:

CCGUCUUUAG UGUGGUGG 18

(2) INFORMATIONS POUR LA SEQ ID NO: 31:

(i) CARACTERISTIQUES DE LA SEQUENCE:
(A) LONGUEUR: 18 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 31:

CUGCAUGUUG GUGUGUGG 18

(2) INFORMATIONS POUR LA SEQ ID NO: 32:

(i) CARACTERISTIQUES DE LA SEQUENCE:
(A) LONGUEUR: 7 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 32:

CUUUGUG 7

(2) INFORMATIONS POUR LA SEQ ID NO: 33:

(i) CARACTERISTIQUES DE LA SEQUENCE:
(A) LONGUEUR: 18 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 33:

CGCGCUUUAG UGUGUGUG 18

(2) INFORMATIONS POUR LA SEQ ID NO: 34:

(i) CARACTERISTIQUES DE LA SEQUENCE:
(A) LONGUEUR: 18 paires de bases

(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 34:

CGCAUCGUGG UUGGUGUG 18

(2) INFORMATIONS POUR LA SEQ ID NO: 35:

(i) CARACTERISTIQUES DE LA SEQUENCE:
(A) LONGUEUR: 18 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 35:

AACUUUUUGG AUAUUGUU 18

(2) INFORMATIONS POUR LA SEQ ID NO: 36:

(i) CARACTERISTIQUES DE LA SEQUENCE:
(A) LONGUEUR: 18 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 36:

AGCUGUUUAG UGUCAGUU 18

(2) INFORMATIONS POUR LA SEQ ID NO: 37:

(i) CARACTERISTIQUES DE LA SEQUENCE:
(A) LONGUEUR: 18 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 37:

CAAUCUUUAG UGUGGUUG 18

(2) INFORMATIONS POUR LA SEQ ID NO: 38:

(i) CARACTERISTIQUES DE LA SEQUENCE:

(A) LONGUEUR: 17 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 38:

GAAGCUUUUG UGACGGU 17

(2) INFORMATIONS POUR LA SEQ ID NO: 39:

(i) CARACTERISTIQUES DE LA SEQUENCE:
(A) LONGUEUR: 19 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 39:

GAAGCGUAUU UGUGACGGU 19

(2) INFORMATIONS POUR LA SEQ ID NO: 40:

(i) CARACTERISTIQUES DE LA SEQUENCE:
(A) LONGUEUR: 17 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 40:

GAAGGGUUUC UGACGGU 17

(2) INFORMATIONS POUR LA SEQ ID NO: 41:

(i) CARACTERISTIQUES DE LA SEQUENCE:
(A) LONGUEUR: 17 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 41:

GAAGCGUGUG UGACGGU 17

(2) INFORMATIONS POUR LA SEQ ID NO: 42:

(i) CARACTERISTIQUES DE LA SEQUENCE:
(A) LONGUEUR: 23 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 42:

GAAGCCUUUN NNAAGGUGAC GGU 23

(2) INFORMATIONS POUR LA SEQ ID NO: 43:

(i) CARACTERISTIQUES DE LA SEQUENCE:
(A) LONGUEUR: 21 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 43:

GAAGCACUGU GUGGUGACGG U 21

(2) INFORMATIONS POUR LA SEQ ID NO: 44:

(i) CARACTERISTIQUES DE LA SEQUENCE:
(A) LONGUEUR: 18 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 44:

GAAGCGUUUU GUGACGGU 18

(2) INFORMATIONS POUR LA SEQ ID NO: 45:

(i) CARACTERISTIQUES DE LA SEQUENCE:
(A) LONGUEUR: 18 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 45:

GAAGCUUUUU GUGACGGU 18

(2) INFORMATIONS POUR LA SEQ ID NO: 46:

(i) CARACTERISTIQUES DE LA SEQUENCE:
(A) LONGUEUR: 18 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 46:

GAAGCAUUAU GUGACGGU 18

(2) INFORMATIONS POUR LA SEQ ID NO: 47:

(i) CARACTERISTIQUES DE LA SEQUENCE:
(A) LONGUEUR: 17 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 47:

GAAGCGUAAG UGACGGU 17

(2) INFORMATIONS POUR LA SEQ ID NO: 48:

(i) CARACTERISTIQUES DE LA SEQUENCE:
(A) LONGUEUR: 19 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 48:

GAAGCCUUCG GGUGACGGU 19

(2) INFORMATIONS POUR LA SEQ ID NO: 49:

(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 49:

CGUCUGUGAA AUUCCGGGGC UUAA 24

(2) INFORMATIONS POUR LA SEQ ID NO: 50:

(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 50:

CGUUUGUGUA AGCNNNCAGC UUAA 24

(2) INFORMATIONS POUR LA SEQ ID NO: 51:

(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 51:

CGUCUGUGAA AUCCCGGGGC UUAA 24

(2) INFORMATIONS POUR LA SEQ ID NO: 52:

(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 52:

CGUCUGUGGA AGUCCGGGGC UUAA 24

(2) INFORMATIONS POUR LA SEQ ID NO: 53:

(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 53:

CGUUUGUGUA AGUCCACGGC UUAA 24

(2) INFORMATIONS POUR LA SEQ ID NO: 54:

(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 54:

CGUCUGUGAA AUUCCACAGC UUAA 24

(2) INFORMATIONS POUR LA SEQ ID NO: 55:

(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 55:

CGUCUGUGUA AUCCAGGGGC UUAA 24

(2) INFORMATIONS POUR LA SEQ ID NO: 56:

(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 56:

CGUUUGUGUA AGUCCACAGC UUAA 24

(2) INFORMATIONS POUR LA SEQ ID NO: 57:

(i) CARACTERISTIQUES DE LA SEQUENCE:
(A) LONGUEUR: 15 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 57:

UAGGUGUGAG GGUCU 15

(2) INFORMATIONS POUR LA SEQ ID NO: 58:

(i) CARACTERISTIQUES DE LA SEQUENCE:
(A) LONGUEUR: 15 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 58:

UAGGUGUGAG UCCCU 15

(2) INFORMATIONS POUR LA SEQ ID NO: 59:

(i) CARACTERISTIQUES DE LA SEQUENCE:
(A) LONGUEUR: 15 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 59:

UAGGUGUAGG GGGCU 15

(2) INFORMATIONS POUR LA SEQ ID NO: 60:

(i) CARACTERISTIQUES DE LA SEQUENCE:
(A) LONGUEUR: 16 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 60:

UAGGUGUGGG UUUCCU 16

(2) INFORMATIONS POUR LA SEQ ID NO: 61:

(i) CARACTERISTIQUES DE LA SEQUENCE:
(A) LONGUEUR: 15 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 61:
UAGGUGUAGG GGACU 15
(2) INFORMATIONS POUR LA SEQ ID NO: 62:
(i) CARACTERISTIQUES DE LA SEQUENCE:
(A) LONGUEUR: 15 paires de bases
(B) TYPE: nucléotide
(C) NOMBRE DE BRINS: simple
(D) CONFIGURATION: linéaire
(ii) TYPE DE MOLECULE: ARNr
(xi) DESCRIPTION DE LA SEQUENCE: SEQ ID NO: 62:
UAGGUGUGGG GAUCU 15
(2) INFORMATIONS POUR LA SEQ ID NO: 63:
(i) CARACTERISTIQUES DE LA SEQUENCE:
(A) LONGUEUR: 15 paires de bases
(B) TYPE: nucléotide
(C) NOMBRE DE BRINS: simple
(D) CONFIGURATION: linéaire
(ii) TYPE DE MOLECULE: ARNr
(xi) DESCRIPTION DE LA SEQUENCE: SEQ ID NO: 63:
UAGGUGUGAG AUCCU 15
(2) INFORMATIONS POUR LA SEQ ID NO: 64:
(i) CARACTERISTIQUES DE LA SEQUENCE:
(A) LONGUEUR: 16 paires de bases
(B) TYPE: nucléotide
(C) NOMBRE DE BRINS: simple
(D) CONFIGURATION: linéaire
(ii) TYPE DE MOLECULE: ARNr
(xi) DESCRIPTION DE LA SEQUENCE: SEQ ID NO: 64:
UAGGUGUGGG GGUUUN 16
(2) INFORMATIONS POUR LA SEQ ID NO: 65:
(i) CARACTERISTIQUES DE LA SEQUENCE:
(A) LONGUEUR: 14 paires de bases
(B) TYPE: nucléotide
(C) NOMBRE DE BRINS: simple
(D) CONFIGURATION: linéaire
(ii) TYPE DE MOLECULE: ARNr

(xi) DESCRIPTION DE LA SEQUENCE: SEQ ID NO: 65:
UAGGUGUGGG GACC 14
(2) INFORMATIONS POUR LA SEQ ID NO: 66:
(i) CARACTERISTIQUES DE LA SEQUENCE:
(A) LONGUEUR: 15 paires de bases
(B) TYPE: nucléotide
(C) NOMBRE DE BRINS: simple
(D) CONFIGURATION: linéaire
(ii) TYPE DE MOLECULE: ARNr
(xi) DESCRIPTION DE LA SEQUENCE: SEQ ID NO: 66:
UAGGUGUAGG GACCU 15
(2) INFORMATIONS POUR LA SEQ ID NO: 67:
(i) CARACTERISTIQUES DE LA SEQUENCE:
(A) LONGUEUR: 16 paires de bases
(B) TYPE: nucléotide
(C) NOMBRE DE BRINS: simple
(D) CONFIGURATION: linéaire
(ii) TYPE DE MOLECULE: ARNr
(xi) DESCRIPTION DE LA SEQUENCE: SEQ ID NO: 67:
UAGGUGUGAG UCCCNU 16
(2) INFORMATIONS POUR LA SEQ ID NO: 68:
(i) CARACTERISTIQUES DE LA SEQUENCE:
(A) LONGUEUR: 15 paires de bases
(B) TYPE: nucléotide
(C) NOMBRE DE BRINS: simple
(D) CONFIGURATION: linéaire
(ii) TYPE DE MOLECULE: ARNr
(xi) DESCRIPTION DE LA SEQUENCE: SEQ ID NO: 68:
UAGGUGUGGG GAUUU 15
(2) INFORMATIONS POUR LA SEQ ID NO: 69:
(i) CARACTERISTIQUES DE LA SEQUENCE:
(A) LONGUEUR: 16 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 69:

GACUUUCGUG CCGUAG 16

(2) INFORMATIONS POUR LA SEQ ID NO: 70:

(i) CARACTERISTIQUES DE LA SEQUENCE:
(A) LONGUEUR: 16 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 70:

GGGGUUCGUG CCGUAG 16

(2) INFORMATIONS POUR LA SEQ ID NO: 71:

(i) CARACTERISTIQUES DE LA SEQUENCE:
(A) LONGUEUR: 16 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 71:

GUCUUCUGUG CCGUAG 16

(2) INFORMATIONS POUR LA SEQ ID NO: 72:

(i) CARACTERISTIQUES DE LA SEQUENCE:
(A) LONGUEUR: 16 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 72:

GAUUUCCGUG CCGUAG 16

(2) INFORMATIONS POUR LA SEQ ID NO: 73:

(i) CARACTERISTIQUES DE LA SEQUENCE:
(A) LONGUEUR: 16 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 73:

GGGUUUUGUG CCGUAG 16

(2) INFORMATIONS POUR LA SEQ ID NO: 74:

(i) CARACTERISTIQUES DE LA SEQUENCE:
(A) LONGUEUR: 16 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 74:

GAUUCCCGUG CCGUAG 16

(2) INFORMATIONS POUR LA SEQ ID NO: 75:

(i) CARACTERISTIQUES DE LA SEQUENCE:
(A) LONGUEUR: 16 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 75:

GGUUUCNGUG CCGUAG 16

(2) INFORMATIONS POUR LA SEQ ID NO: 76:

(i) CARACTERISTIQUES DE LA SEQUENCE:
(A) LONGUEUR: 16 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 76:

GUUUUCUGUG CCGUAG 16

(2) INFORMATIONS POUR LA SEQ ID NO: 77:

(i) CARACTERISTIQUES DE LA SEQUENCE:
(A) LONGUEUR: 16 paires de bases
(B) TYPE: nucléotide
(C) NOMBRE DE BRINS: simple

(D) CONFIGURATION: linéaire

(ii) TYPE DE MOLECULE: ARNr

(xi) DESCRIPTION DE LA SEQUENCE: SEQ ID NO: 77:

GGUUUCUGUG CCGUAG 16

(2) INFORMATIONS POUR LA SEQ ID NO: 78:

(i) CARACTERISTIQUES DE LA SEQUENCE:
(A) LONGUEUR: 16 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 78:

GGGGUUUGUG CCGUAG 16

(2) INFORMATIONS POUR LA SEQ ID NO: 79:

(i) CARACTERISTIQUES DE LA SEQUENCE:
(A) LONGUEUR: 16 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 79:

GGGAUUUGUG CCGUAG 16

(2) INFORMATIONS POUR LA SEQ ID NO: 80:

(i) CARACTERISTIQUES DE LA SEQUENCE:
(A) LONGUEUR: 20 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 80:

UGCAGGAUCG GCGUAGUGAU 20

(2) INFORMATIONS POUR LA SEQ ID NO: 81:

(i) CARACTERISTIQUES DE LA SEQUENCE:
(A) LONGUEUR: 20 paires de bases
(B) TYPE: nucléotide

(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 81:

CACCAGAUCG CCGUAGAGAU 20

(2) INFORMATIONS POUR LA SEQ ID NO: 82:

(i) CARACTERISTIQUES DE LA SEQUENCE:
(A) LONGUEUR: 20 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 82:

UACAGGAUCG CGCCAGAGAU 20

(2) INFORMATIONS POUR LA SEQ ID NO: 83:

(i) CARACTERISTIQUES DE LA SEQUENCE:
(A) LONGUEUR: 20 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 83:

UACAGGAUCG CUGCAGAGAU 20

(2) INFORMATIONS POUR LA SEQ ID NO: 84:

(i) CARACTERISTIQUES DE LA SEQUENCE:
(A) LONGUEUR: 20 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 84:

GGCAGGACCG GCGUGGAGAC 20

(2) INFORMATIONS POUR LA SEQ ID NO: 85:

(i) CARACTERISTIQUES DE LA SEQUENCE:
(A) LONGUEUR: 20 paires de bases

(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 85:

CACUGGAUUG CCAUGGAGAC 20

(2) INFORMATIONS POUR LA SEQ ID NO: 86:

(i) CARACTERISTIQUES DE LA SEQUENCE:
(A) LONGUEUR: 20 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 86:

UGCAGGAUUG GGUCAGAGAU 20

(2) INFORMATIONS POUR LA SEQ ID NO: 87:

(i) CARACTERISTIQUES DE LA SEQUENCE:
(A) LONGUEUR: 21 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 87:

CACCAGACGG UCGUAGAGAU A 21

(2) INFORMATIONS POUR LA SEQ ID NO: 88:

(i) CARACTERISTIQUES DE LA SEQUENCE:
(A) LONGUEUR: 20 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 88:

CACUAGAUCG CUGUAGAGAU 20

(2) INFORMATIONS POUR LA SEQ ID NO: 89:

(i) CARACTERISTIQUES DE LA SEQUENCE:

(A) LONGUEUR: 20 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 89:

CACCGGAUCG GGCUAGAGAU 20

(2) INFORMATIONS POUR LA SEQ ID NO: 90:

(i) CARACTERISTIQUES DE LA SEQUENCE:
(A) LONGUEUR: 20 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 90:

UACGGGAUCG CCGCAGAGAU 20

(2) INFORMATIONS POUR LA SEQ ID NO: 91:

(i) CARACTERISTIQUES DE LA SEQUENCE:
(A) LONGUEUR: 20 paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 91:

UACAAGACAG GCGUAGAGAU 20

(2) INFORMATIONS POUR LA SEQ ID NO: 92:

(i) CARACTERISTIQUES DE LA SEQUENCE:
(A) LONGUEUR:  paires de bases
(B) TYPE: nucléotide
(C) NOMBRE DE BRINS: simple
(D) CONFIGURATION: linéaire

(ii) TYPE DE MOLECULE: ARNr

(xi) DESCRIPTION DE LA SEQUENCE: SEQ ID NO: 92:
CGCTGGCGGC GTGCTTAACA CATGCAAGTC GAACGGAAAG GCCTAGCTTG CTAGGTACTC 60
GAGTGGCGAA CGGGTGAGTA ACACGTGGGT GATCTGCCTC GTACTTCGGG ATAAGCCTGG 120
GAAACTGGGT CTAATACTGG ATAGGACCAT GCTTTAGTGT GTGTGGTGGA AAGTTTTTCG 180
GTACGAGATG AGCCCGCGGC CTATCAGCTT GTTGGTGGGG TAATGGCCTA CCAAGGCGTC 240
GACGGGTAGC CGGCCTGAGA GGGTGGACGG CCACATTGGG ACTGAGATAC GGCCCAGACT 300
CCTACGGGAG GCAGCAGTGG GGAATATTGC ACAATGGGCG CAAGCCTGAT GCAGCGACGC 360
CGCGTGGGGG ATGACGGCCT TCGGGTTGTA AACCTCTTTC GCTAGGGACG AAGCTTTTGT 420
GACGGTACCT AGATAAGAAG CACCGGCTAA CTACGTGCCA GCAGCCGCGG TAATACGTAG 480
GGTGCGAGCG TTGTCCGGAA TTACTGGGCG TAAAGAGCTC GTAGGTGGTT TGTCGCGTCG 540
TCTGTGAAAT TCCGGGGCTT AACTTCGGGC GTGCAGGCGA TACGGGCATA ACTTGAGTGC 600
TGTAGGGGAG ACTGGAATTC CTGGTGTAGC GGTGGAATGC GCAGATATCA GGAGGAACAC 660
CGATGGCGAA GGCAGGTCTC TGGGCAGTAA CTGACGCTGA GGAGCGAAAG CATGGGGAGC 720
GAACAGGATT AGATACCCTG GTAGTCCATG CCGTAAACGG TGGGCGCTAG GTGTGAGGGT 780
CTTCCACGAC TTTCGTGCCG TAGCTAACGC ATTAAGCGCC CCGCCTGGGG AGTACGGCCG 840
CAAGGCTAAA ACTCAAAGGA ATTGACGGGG GCCCGCACAA GCGGCGGAGC ATGTGGATTA 900
ATTCGATGCA ACGCGAAGAA CCTTACCTGG GCTTGACATA TGCAGGATCG GCGTAGTGAT 960
ACGTTTTCCC TTGTGGTCTG TATACAGGTG GTGCATGGTT GTCGTCAGCT CGTGTCGTGA 1020
GATGTTGGGT TAAGTCCCGC AACGAGCGCA ACCCTTGTCT TATGTTGCCA GCACGTGATG 1080
GTGGGGACTC ATGAGAGACT GCCGGGGTTA ACTCGGAGGA AGGTGGGGAT GACGTCAAAT 1140
CATCATGCCC CTTATGTCCA GGGCTTCACA CATGCTACAA TGGTCGGTAC AACGCGCTGC 1200
GAGCCTGTGA GGGTGAGCGA ATCGCTGAAA GCCGGCCTCA GTTCGGATTG GGGTCTGCAA 1260
CTCGACCCCA TGAAGTCGGA GTCGCTAGTA ATCGCAGATC AGCAACGCTG CGGTGAATAC 1320
GTNCCCGGGC CTTGTACACA CCGCCCGTCA CGTCATGAAA GTTGGTAACA CCCGAAGCCA 1380
GTGGCCTAAC CCTTGTGGGG GGGAGCTGTC GAAGGTGGNA TCGGCGATTG GNACGAAGTC 1440
GTAACAAGGT AGCCGTACCG GAAGGTGCGG GCTGG 1475